# EUROPEAN PATENT APPLICATION

(11) **EP 3 974 028 A1**
(43) Date of publication of application: **30.03.2022**
(21) Application number: 20810161.8
(22) Date of filing: 13.05.2020
(51) Int. Cl.: A61P 19/02, C07K 14/00, A61K 38/16, A61L 27/22, A61L 27/46, A61L 27/52, A61L 27/54

(54) **PHARMACEUTICAL COMPOSITION FOR JOINT DISEASE TREATMENT AND METHOD FOR PRODUCING SAME**

(30) Priority: 17.05.2019 JP 2019094073
(71) Applicant: Educational Foundation Of Osaka Medical And Pharmaceutical University, Takatsuki-shi, Osaka 569-8686 (JP); The School Corporation Kansai University, Suita-shi, Osaka 564-8680 (JP)
(72) Inventor: OTSUKI Shuhei, Takatsuki City, Osaka 5698686 (JP); OKUNO Nobuhiro, Takatsuki City, Osaka 5698686 (JP); HIRANO Yoshiaki, Suita-shi, Osaka 564-8680 (JP)
(74) Representative: Greaves Brewster LLP
(86) International application number: PCT/JP2020/019083
(87) International publication number: WO 2020/235412

(57) **Abstract**

The pharmaceutical composition according to the present invention is a pharmaceutical composition for treating joint diseases comprising a peptide hydrogel constituted of a self-assembled peptide, wherein the peptide contains a β-hairpin structure constituted of β-sheet structures composed of alternating sequences of hydrophilic amino acids and hydrophobic amino acids, and a β-turn structure having biological activity.

## Description

### TECHNICAL FIELD

The present invention relates to a pharmaceutical composition for treating joint diseases and a method for producing the same, and more particularly to a pharmaceutical composition for treating joint diseases containing a peptide hydrogel and a method for producing the same.

### BACKGROUND ART

Traditionally, in the field of tissue engineering, it has been considered that living tissues are regenerated by three elements: cells, growth factors, and scaffolding materials. The conditions for an effective scaffolding material are the formability of a three-dimensional structure, biodegradability, cell adhesion, and mechanical strength that mimics the surrounding environment. In recent years, hydrogels made of animal-derived proteins such as collagen gel and gelatin gel have been used as scaffolding materials satisfying these conditions. However, since these protein gels are extracts derived from animals, there is a possibility of a difference in composition between lots, and an infectious disease when considering medical applications such as regenerative medicine and the like.

Then, in order to solve such a problem, it is considered to use a peptide hydrogel composed of a peptide that can be artificially synthesized as a scaffolding material. It is known that a peptide composed of a repeating sequence of hydrophilic amino acids and hydrophobic amino acids forms a β-sheet (β-strand) structure by the repeating sequence, and self-assembly occurs by adding a salt to the peptide, and a hydrogel is formed by encapsulating water at that time.

The peptide hydrogel formed as described above can solve the problem of the hydrogel composed of the above-mentioned animal-derived protein, and is considered to be effective for medical applications such as regenerative medicine and the like. In fact, it is known that peptide hydrogels can be transplanted into bone defects to provide a good scaffold for cells necessary for bone regeneration and promote bone regeneration, and in particular, it is presented in Patent Literatures 1 and 2 that peptide hydrogels are applied to regenerative medicine of bone tissue and periodontal tissue. Specifically, Patent Literature 1 discloses that the formation of new bone was recognized by directly transplanting the peptide hydrogel into the bone defect formed in the alveolar bone of a dog. Further, Patent Literature 2 discloses that transplantation of the peptide hydrogel into a bone defect formed in the skull bone of a rat promotes bone regeneration.

Further, it is disclosed in Non-Patent Literatures 1 and 2 that since the mechanical strength that imitates the surrounding environment is required as the condition for an effective scaffolding material as described above, the peptide hydrogel is required to have the same mechanical strength (rigidity) as the surrounding environment of the transplant site, however, the conventional peptide hydrogels have low mechanical strength, and the strength needs to be improved.

### CITATION LIST

### PATENT LITERATURE

[Patent Literature 1] Japanese Laid-Open Patent Publication No. 2007-105186
[Patent Literature 2] Japanese Laid-Open Patent Publication (Translation of PCT Application) No. 2010-504972

### NON-PATENT LITERATURE

[Non-Patent Literature 1] Sotirios Koutsopoulos., Journal of Biomedical Materials Research A, 2016, 104 (4), pp. 1002-1016.
[Non-Patent Literature 2] Jiaju Lu and Xiumei Wang, Biomimetic Medical Materials (Part of the Advances in Experimental Medicine and Biology book series (AEMB, volume 1064)), 2018, pp. 297-312.

### SUMMARY OF INVENTION

### TECHNICAL PROBLEM

As described in Non-Patent Literatures 1 and 2, it is necessary to increase the rigidity in order to apply the peptide hydrogel as a material for transplantation into a living tissue, and various studies have been conducted so far, however, the rigidity of the peptide hydrogel has not been sufficiently increased and a gel having rigidity relatively close to that of a liquid is used in the present circumstances. Therefore, especially when transplanting to bone or its surroundings, the above-mentioned effective scaffolding conditions such as mechanical strength imitating the surrounding environment cannot be sufficiently satisfied. Further, when such a gel is transplanted to a desired site in a living body, the gel does not stay at the desired site but flows to the surroundings, so that a high tissue regeneration effect at the desired site cannot be expected. In Patent Literatures 1 and 2 above, because a relatively deep bone defect is prepared in the bone and the gel is filled therein, a certain amount of gel can be expected to remain in the bone defect even if the gel has low rigidity, however, it cannot be said that a sufficient effect can be obtained for actual tissue defects in various tissues.

Further, the bone tissue disclosed in Patent Literatures 1 and 2 is a tissue having relatively good blood circulation, and is advantageous for tissue regeneration because cells and growth factors easily gather, however, there is a demand for regenerative therapy that exhibits an excellent tissue regeneration effect even in sites that are in an environment having poor blood circulation and unfavorable for tissue regeneration such as, for example, joints and the like.

The present invention has been made in view of the above problems, and an object of the present invention is to obtain a pharmaceutical composition containing a peptide hydrogel suitable for tissue regeneration of an affected area in a joint disease by improving the mechanical strength of the peptide hydrogel.

### SOLUTION TO PROBLEM

The present inventors have intensively studied in order to achieve the above object and resultantly found that a peptide hydrogel having a higher mechanical strength than the conventional one can be fabricated by self-assembling a peptide having a β-hairpin structure containing a β-turn structure having biological activity, and further that tissue regeneration can be promoted by transplanting the peptide hydrogel into a defect in joint tissue, leading to completion of the present invention.

Specifically, the pharmaceutical composition according to the present invention is for the treatment of joint diseases and contains a peptide hydrogel constituted of a self-assembled peptide, characterized in that the peptide contains a β-hairpin structure constituted of a β-sheet structure composed of alternating sequences of hydrophilic amino acids and hydrophobic amino acids and a β-turn structure having biological activity.

According to the pharmaceutical composition according to the present invention, since the peptide constituting the peptide hydrogel has a β-hairpin structure, the bonding point between the peptides (intramolecular hydrogen bond, etc.) can be made larger than that of the peptide hydrogel constituted of the peptide having β-sheet structures and can increase the mechanical strength (rigidity). Hence, when the peptide hydrogel according to the present invention is transplanted into the joint tissue, it becomes difficult to flow around the desired transplantation site and can be relatively maintained at the site, which is extremely effective in promoting tissue regeneration. In addition, since the peptide contains a β-turn having biological activity, various biological activities can be imparted to the peptide hydrogel, and for example, when the β-turn consists of a sequence showing cell adhesion activity, cell adhesion activity can be imparted to the peptide hydrogel, and interaction with cells is improved, thus, the function as a scaffolding material can be improved. Owing to the above effect, the pharmaceutical composition according to the present invention can exhibit an action capable of promoting tissue regeneration even in joint tissue in an environment in which blood circulation is poor and tissue regeneration is disadvantageous.

In the pharmaceutical composition according to the present invention, the peptide hydrogel preferably has a difference between the storage elastic modulus G' and the loss elastic modulus G" of 3900 Pa or more.

In this way, the mechanical strength of the peptide hydrogel is increased, and the above effect can be further improved.

In the pharmaceutical composition according to the present invention, the peptide is preferably composed of an amino acid sequence of IKIKIKIKIKRGDSKIKIKIKIKI (SEQ ID NO: 1).

In the pharmaceutical composition according to the present invention, the joint disease is preferably meniscus injury.

The method for producing a pharmaceutical composition according to the present invention is a method for producing the pharmaceutical composition according to the present invention, characterized by comprising a step of preparing an aqueous solution containing the peptide, and a step of adding phosphate buffered saline (PBS) to the aqueous solution to generate a peptide hydrogel.

The present inventors have found that by adding various concentrations of PBS to an aqueous solution of a peptide having the above-mentioned characteristics, the mechanical strength of the peptide hydrogel produced in a concentration-dependent manner can be improved. That is, according to the method for producing a pharmaceutical composition according to the present invention, the peptide hydrogel can be easily prepared, and further, the mechanical strength of the peptide hydrogel can be adjusted by the concentration of PBS.

In the method for producing a pharmaceutical composition according to the present invention, the phosphate buffered saline is preferably added to the aqueous solution so that the final concentration is 3 times the concentration (3 × PBS) or more.

In this way, a peptide hydrogel having a large mechanical strength with a difference between the storage elastic modulus G' and the loss elastic modulus G" of 3900 Pa or more can be obtained.

It is preferable that the method for producing a pharmaceutical composition according to the present invention further comprises a step of adjusting the concentration of the phosphate buffered saline in order to adjust the optimum elastic modulus of the peptide hydrogel depending on to the site to which the pharmaceutical composition according to the present invention is applied.

### ADVANTAGEOUS EFFECTS OF INVENTION

According to the pharmaceutical composition according to the present invention and the method for producing the same, it is possible to obtain a pharmaceutical composition which contains a peptide hydrogel having a high mechanical strength, can be maintained at a desired site when transplanted into a living tissue, and exhibits an excellent effect of promoting tissue regeneration.

### BRIEF DESCRIPTION OF DRAWINGS

FIG. 1 is a graph showing the storage elastic modulus G' (left bar) and the loss elastic modulus G" (right bar) of peptide hydrogels obtained using EMEM, 5 wt% NaCl aqueous solution and 5 × PBS, respectively.
FIG. 2 is a graph showing the storage elastic modulus G' (left bar) and loss elastic modulus G" (right bar) of peptide hydrogels obtained using 5 wt% NaCl aqueous solution, 1 × PBS, 3 × PBS and 5 × PBS, respectively.
FIG. 3(a) is a photograph showing a defect in the meniscus of a rabbit prepared in the present example, and FIG. 3(b) is a photograph showing a peptide hydrogel used for transplantation in the present example.
FIG. 4 provides photographs for explaining Quantitative Analysis of the Occupation Ratio of Tissue Regeneration (R/D ratio) performed in the present example.
FIG. 5 provides photographs showing the meniscus after 2, 4, 8 and 12 weeks in the case where a peptide hydrogel was transplanted into the rabbit meniscus defect (KI24RGDS group) and where it was not transplanted (Control group), respectively.
FIG. 6 provides photographs showing safranin O-stained meniscus tissue at 2, 4, 8 and 12 weeks after a peptide hydrogel was transplanted into the meniscus defect of a rabbit (KI24RGDS group).
FIG. 7 provides photographs showing safranin O-stained meniscus tissue at 2, 4, 8 and 12 weeks after the preparation of the defect in the meniscus defect of a rabbit (control group).
FIG. 8 is a graph showing the results of Ishida Score of the KI24RGDS group and the control group in the present example.
FIG. 9 is a graph showing the results of Quantitative Analysis of the Occupation Ratio of Tissue Regeneration (R/D ratio) of the KI24RGDS group and the control group in the present example.
FIGS. 10(a) to 10(c) provide photographs and a model diagram showing the meniscus tissue 2 weeks after a peptide hydrogel was transplanted into the meniscus defect of a rabbit, and FIG. 10(a) is a HE-stained photograph, FIG. 10(b) is a safranin O-stained photograph, and FIG. 10(c) is a model diagram showing the state of tissue regeneration.

### DESCRIPTION OF EMBODIMENTS

Hereinafter, embodiments for carrying out the present invention will be described with reference to the drawings. The following description of preferred embodiments is merely exemplary and is not intended to limit the invention, its application methods or its uses.

The pharmaceutical composition according to the present invention contains a peptide hydrogel constituted of self-assembled peptides, and is particularly a pharmaceutical composition used for the treatment of joint diseases. Further, in the pharmaceutical composition according to the present invention, the peptide is characterized by containing a β-hairpin structure constituted of β-sheet structures composed of alternating sequences of hydrophilic amino acids and hydrophobic amino acids and a β-turn structure having biological activity.

In the pharmaceutical composition according to one embodiment of the present invention, the peptide hydrogel is a hydrogel obtained by encapsulating water when a peptide having a structure having both a hydrophilic surface and a hydrophobic surface is self-assembled under predetermined conditions. Further, the peptide constituting the peptide hydrogel is a peptide having a β hairpin structure formed of β sheets and a β turn located between the β sheets and connecting them as described above. The β sheet is formed by alternating sequences of hydrophilic amino acids and hydrophobic amino acids as described above, and has both a hydrophilic surface and a hydrophobic surface. In the present embodiment, the β sheet is preferably formed by repeating sequences of lysine (K) and isoleucine (I).

The β-turn usually refers to a turn structure stabled due to hydrogen bond formation between the carbonyl oxygen atom of the n-th amino acid residue and the amide proton of the n+3-th amino acid residue. There are various amino acid sequences that form β-turns, which are well known to those of skill in the art. In the present embodiment, the amino acid sequence forming a β-turn has biological activity, and the biological activity is preferably cell adhesion activity. In the present embodiment, the β-turn preferably consists of an arginine-glycine-aspartic acid-serine (RGDS) sequence. The RGDS sequence is known as a sequence forming a β-turn, and further, the RGDS sequence is a sequence derived from fibronectin and is known to bind to integrin αVβI and exhibit cell adhesion activity.

In the present embodiment, the β hairpin structure is formed by shielding the electrostatic repulsion of the hydrophilic surface (lysine residue) of the β sheet by the action of a salt on the peptide having the above sequence. The β-hairpin structure is stably formed by intramolecular hydrophobic interaction between side chains and an intramolecular hydrogen bond between the carbonyl and amide moieties of the peptide backbone, which facilitates self-assembly.

In particular, in the present embodiment, the β hairpin structure is preferably a β hairpin structure in which the RGDS sequence is arranged between the repeating sequences of K and I. The number of repetitions of K and I in the β sheet is not particularly limited, but for example, the number of repetitions is preferably 5, and therefore, the amino acid sequence forming the β hairpin structure in the present embodiment is preferably IKIKIKIKIKRGDSKIKIKIKIKI (KI24RGDS: SEQ ID NO: 1).

In the present embodiment, the peptide hydrogel preferably has high mechanical strength. Specifically, it is preferable that the peptide hydrogel has a mechanical strength that makes it difficult for the peptide hydrogel to flow to the surroundings at a desired transplanted portion in a living body, and in particular, the difference between the storage elastic modulus G' and the loss elastic modulus G" is preferably 3900 Pa or more, and more preferably 10000 Pa or more.

In the pharmaceutical composition according to the present embodiment, the joint disease refers to a disease in which an abnormality such as deformation, injury or inflammation occurs in the joint. In the present embodiment, the joint refers to, but not limited to, a portion where bones are connected to each other in parts such as shoulders, elbows, fingers, crotch, knees and ankles. The joint disease in the present embodiment is specifically a disease of a part of the joint other than the bone, and includes, for example, diseases of cartilage, ligament, and meniscus.

The pharmaceutical compositions according to the present embodiment is used transplanted into the affected area of a joint disease, and more specifically is implanted into the defect of the site of a joint disease. The defect may be a portion that is defective due to the disease itself, or may be a portion that is created by active excision by surgery.

The pharmaceutical composition according to the present embodiment may contain various pharmaceutically acceptable additives in addition to the peptide hydrogel. However, it is preferable that the additive does not reduce the mechanical strength of the peptide hydrogel and does not reduce the mechanical strength of the pharmaceutical composition.

The method for producing a pharmaceutical composition according to the present invention is a method including the preparation of a peptide hydrogel as described above, and specifically, comprises a step of preparing an aqueous solution containing a peptide and and a step of adding phosphate buffered saline (PBS) to the aqueous solution to generate a peptide hydrogel.

In the method for producing a pharmaceutical composition according to an embodiment of the present invention, the peptide and the peptide hydrogel are a peptide and a peptide hydrogel having the above-mentioned characteristics, respectively. The peptide having a desired sequence can be synthesized by a method well known to those skilled in the art, for example, by using a well-known Fmoc solid-phase synthesis method. The synthesized peptide may be purified by a well-known method such as high performance liquid chromatography (HPLC) or the like. Then, the obtained peptide can be dissolved in water to prepare an aqueous peptide solution. The peptide concentration of the aqueous solution is not particularly limited, but can be adjusted to, for example, about 1 to 10%.

In the present embodiment, a peptide hydrogel is obtained by adding PBS to the aqueous solution prepared as described above. Specifically, the salt in PBS shields the electrostatic repulsion of the hydrophilic surface of the peptide to form a β-hairpin structure, which becomes stable by the intramolecular hydrophobic interaction between side chains of the peptide and an intramolecular hydrogen bond between the carbonyl and amide moieties of the peptide backbone, facilitating self-assembly. At this time, water in the aqueous solution is encapsulated, and as a result, a peptide hydrogel is generated. When PBS is added to an aqueous solution containing a peptide, it is preferable to add an equal amount of PBS to the aqueous solution. In this case, PBS at a concentration twice the desired final concentration of PBS will be added.

In the present embodiment, the concentration of PBS is not particularly limited, but the mechanical strength of the resultant peptide hydrogel can be increased by increasing the concentration of PBS. However, if the concentration of PBS is increased excessively, salt precipitation may occur, so it is not preferable to increase the concentration excessively. In the present embodiment, for example, it is preferable to mix 10 × PBS with the peptide-containing aqueous solution in an equal amount to obtain a final concentration of 5 × PBS. In addition, 1 × PBS can be prepared by a well-known method, for example, it can be prepared by adding 1 L of water to 8 g of sodium chloride (NaCI), 0.2 g of potassium chloride (KCl), 1.44 g of disodium hydrogen phosphate and 0.24 g of potassium dihydrogen phosphate, and adjusting pH to 7.4 with hydrochloric acid or the like. Therefore, 2 × PBS can be prepared by doubling the amount of each of the above salts, and 10 × PBS can be prepared by multiplying the amount of each of the above salts by 10 times. As described above, since the mechanical strength of the resultant peptide hydrogel can be increased by increasing the concentration of PBS, the mechanical strength of the peptide hydrogel can be be adjusted by adjusting the concentration of PBS used.

### EXAMPLES

Hereinafter, examples for explaining in detail the pharmaceutical composition according to the present invention and the method for producing the same are shown.

[Example 1: Preparation of peptide hydrogel] First, in order to prepare the peptide hydrogel according to the present example, a peptide consisting of an amino acid sequence of IKIKIKIKIKRGDSKIKIKIKIKI (KI24RGDS: SEQ ID NO: 1) was synthesized by using the Fmoc solid-phase synthesis method, which is a conventional method for peptide synthesis. The synthesized peptide was isolated and purified using well-known HPLC.

Six milligrams (6 mg) of the KI24RGDS peptide obtained as described above was dissolved in 100 µL of ultrapure water to prepare a 6% aqueous peptide solution.

Next, an equal amount (100 µL) of 2 × PBS, 4 × PBS, 6 × PBS or 10 × PBS was added to the aqueous peptide solution and mixed. Therefore, the final concentration of PBS in the mixture was 1 × PBS, 2 × PBS, 3 × PBS, and 5 × PBS, respectively. Thereafter, it was allowed to stand still in a cool and dark place for one day. As a result, the peptide in the aqueous peptide solution self-assembled by the action of the salt in PBS, and a peptide hydrogel was obtained. In addition, as a comparative example, Eagle's minimum essential medium (EMEM), which contains salts necessary for gel formation but is considered to be toxic in vivo, or 10 wt% NaCl aqueous solution (final concentration is 5 wt%) was added in an equal amount to the peptide aqueous solution and mixed, and the mixture was allowed to stand still in a cool and dark place for 1 day. A peptide hydrogel was also obtained in this comparative example.

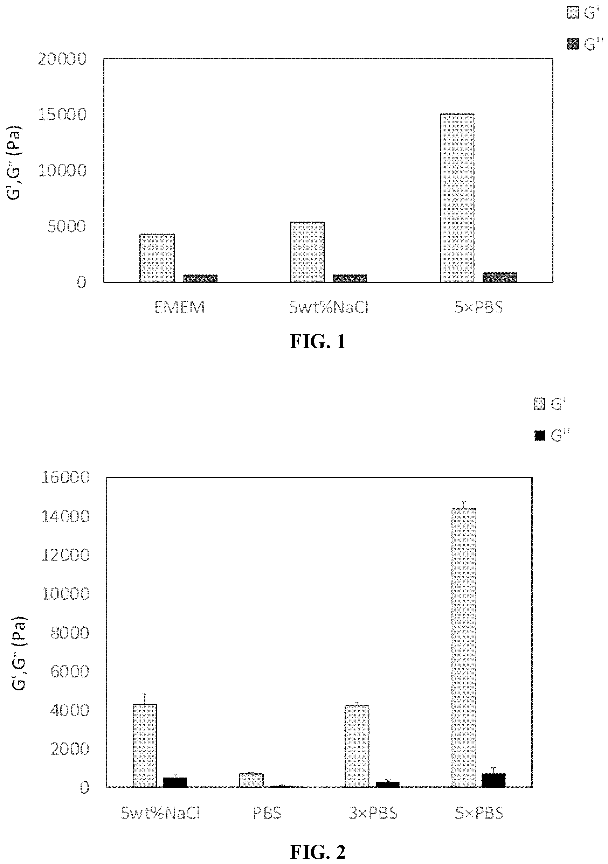

[Example 2: Measurement of mechanical strength of peptide hydrogel] In order to evaluate the mechanical strength of each peptide hydrogel obtained as described above, a rheometer (HAAKE viscosity/viscoelasticity measuring device (HAAKE MARS40) manufactured by Thermo Scientific) was used for each peptide hydrogel and the rheology thereof was measured. FIGS. 1 and 2 show the storage elastic modulus G' and the loss elastic modulus G" of each peptide hydrogel obtained by the rheology measurement. Specifically, FIG. 1 shows a graph comparing the measurement results of peptide hydrogels produced by EMEM, 5 wt% NaCl, 5 × PBS using a peptide of KI24RGDS, and FIG. 2 shows a graph comparing the measurement results of peptide hydrogels produced by EMEM, 1 × PBS, 2 × PBS, 3 × PBS, 5 × PBS using a peptide of KI24RGDS.

As shown in FIG. 1, when EMEM or a 5 wt% NaCl aqueous solution was used as the salt for producing the peptide hydrogel, the storage elastic modulus G' of the peptide hydrogel was about 5000 and the loss elasticity modulus G" was about 600 in any case. On the other hand, when 5 × PBS was used as the salt, the storage elastic modulus G' of the peptide hydrogel was about 15,000, and the loss elastic modulus G" was about 800. Therefore, it became clear that the difference between the storage elastic modulus G' and the loss elastic modulus G" was larger and a peptide hydrogel having higher mechanical strength with higher rigidity could be obtained when 5 × PBS was used than when EMEM or 5 wt% NaCl aqueous solution was used as the salt.

Further, as shown in FIG. 2, as the concentration of PBS used for the production of the peptide hydrogel was increased, the difference between the storage elastic modulus G' and the loss elastic modulus G" was increased, that is, a peptide hydrogel having higher mechanical strength with higher rigidity was obtained. If compared with the case of using a 5 wt% NaCl aqueous solution, the difference between the storage elastic modulus G' and the loss elastic modulus G" was larger (approximately 3950 for 3 × PBS and approximately 3750 for 5 wt% NaCl aqueous solution) and a peptide hydrogel having higher mechanical strength was obtained when PBS having a concentration of 3 × PBS or higher was used than when a 5 wt% NaCl aqueous solution was used. From the above results, it became clear that the rigidity of the peptide hydrogel obtained could be improved depending on the concentration of PBS used.

[Example 3: Application of peptide hydrogel to rabbit meniscus] Next, in order to examine the meniscus regeneration effect by transplanting the peptide hydrogel into the meniscus defect, the effect was evaluated using a rabbit meniscus defect model. The method and results will be described below.

### (Method)

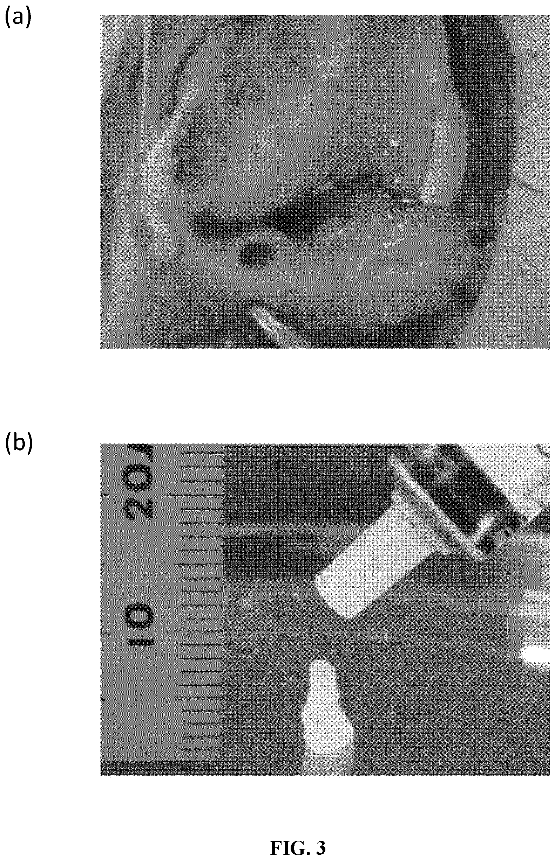

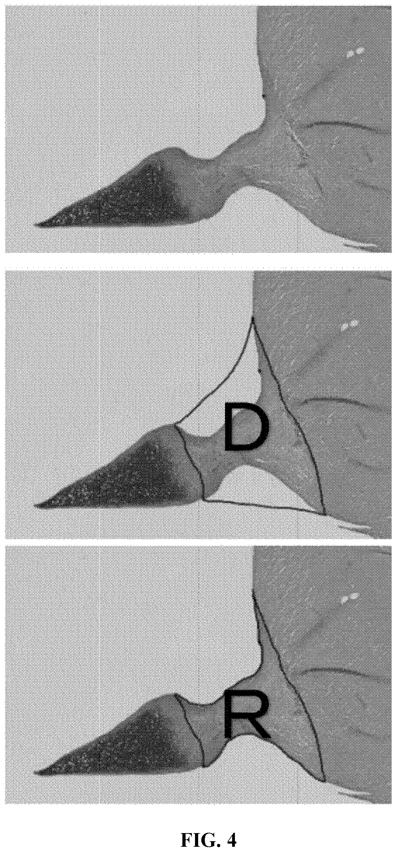

First, 20 male Japanese white rabbits weighing 3.0 to 3.4 kg at 6 months of age were prepared, and a cylindrical defect with a diameter of 2.0 mm was prepared in the medial anterior segment of the meniscus of both knees (FIG. 3A). Further, a peptide hydrogel produced by the above method using a peptide of KI24RGDS and 5 × PBS was prepared (FIG. 3B), and the peptide hydrogel was filled in the defect. The group in which the defect was filled with peptide hydrogel was designated as the KI24RGDS group, and the group in which no treatment was applied to the defect was designated as the control group. After transplantation, tissue evaluation was performed on the knees of each group using hematoxylin/eosin staining (HE staining) or safranin O staining. Tissue evaluation was performed at 2, 4, 8 and 12 weeks after transplantation, and 5 animals (10 knees) were evaluated at each evaluation period. In addition, the tissue evaluation used Ishida score and Quantitative Analysis of the Occupation Ratio of Tissue Regeneration (R/D ratio). In the evaluation based on Ishida score, scores were given from the three viewpoints shown in Table 1 below, and the degree of tissue repair was evaluated by the total. On the other hand, in the evaluation based on the R/D ratio, the degree of tissue repair was evaluated by analyzing the area of the entire defect (D) and the area of the repaired tissue (R) and calculating the ratio of R/D using ImageJ software as shown in FIG. 4.

**[Table 1]**

| 1. Connection of repaired tissue | |
|---|---|
| 2 point | Connected to surrounding meniscus on both sides |
| 1 point | Partially connected to surrounding meniscus |
| 0 point | No connection to surrounding meniscus |

| 2. Presence of fibrocartilage cell | |
|---|---|
| 2 point | Widespread presence of fibrocartilage cell in repaired tissue |
| 1 point | Local presence of fibrocartilage cell in repaired tissue |
| 0 point | No fibrocartilage cell present in repaired tissue |

| 3. Safranin O-staining | |
|---|---|
| 2 point | Deeply stained with safranin O |
| 1 point | Slightly stained with safranin O |
| 0 point | Not stained with safranin O |

### (Result)

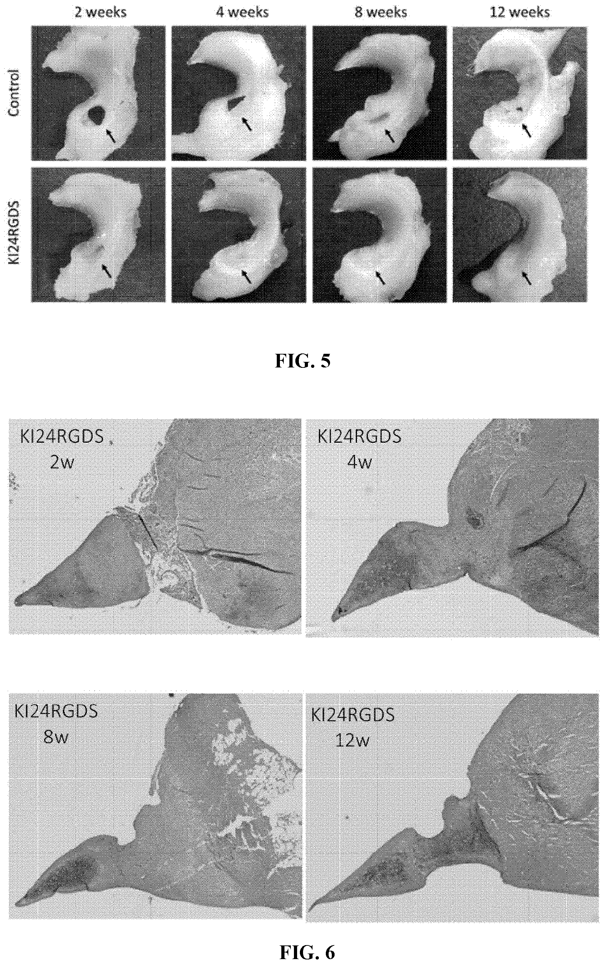

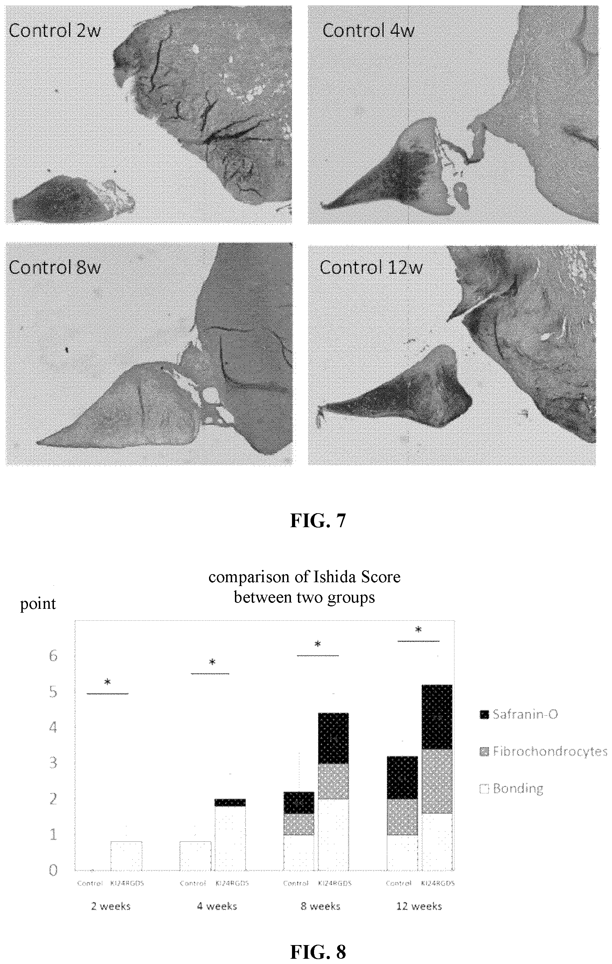

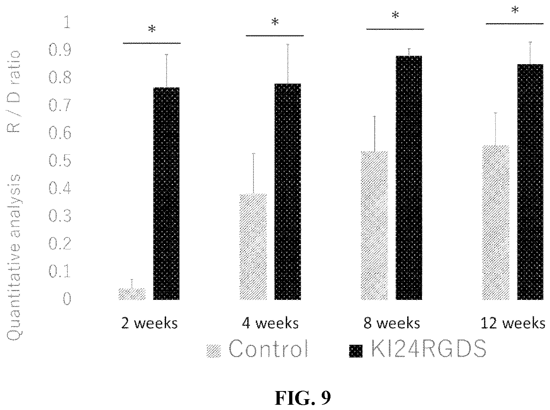

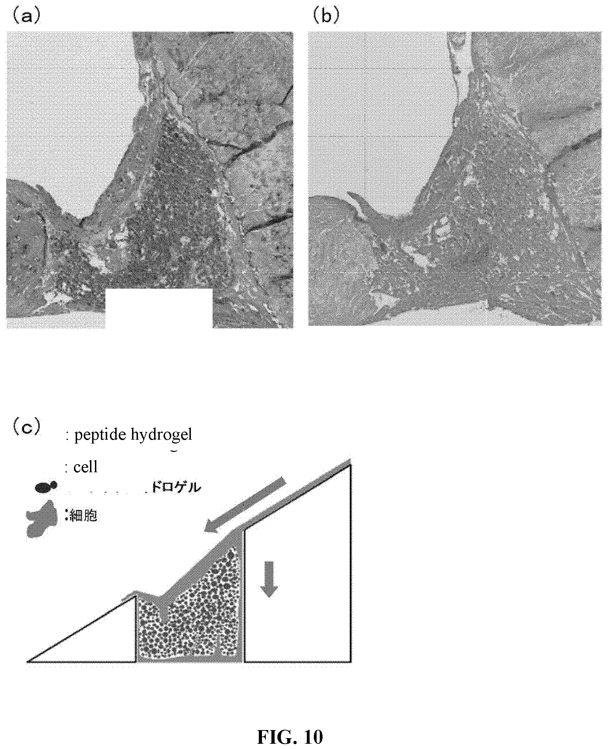

FIG. 5 shows photographs of the meniscus at 2, 4, 8 and 12 weeks after transplantation of the KI24RGDS group and the control group, FIG. 6 shows photographs of the safranin O-stained meniscus defect at 2, 4, 8 and 12 weeks after transplantation of the KI24RGDS group, and FIG. 7 shows photographs of the safranin O-stained meniscus defect at 2, 4, 8 and 12 weeks after the preparation of the defect in the control group. In addition, FIG. 8 shows the results of Ishida scores in each group, and FIG. 9 shows the results of the R/D ratio. Furthermore, FIGS. 10A to 10C shows photographs of the meniscus defect stained with HE and safranin O at 2 weeks after transplantation of the KI24RGDS group, and a model diagram of the process of tissue regeneration by the peptide hydrogel.

As shown in FIG. 5, it is clear in the macroscopic findings of the meniscus that in the KI24RGDS group, the defect (part indicated by the arrow in the figure) became smaller and repaired as 2, 4, 8 and 12 weeks after transplantation. On the other hand, in the control group, although the defect became smaller as 2, 4, 8 and 12 weeks after the preparation of the defect, the degree of repair was clearly lower than that in the KI24RGDS group, and even after 12 weeks, the presence of holes penetrating the meniscus was observed. Also, in the photograph of the meniscus tissue stained with safranin O, it is clear as shown in FIG. 6 that in the KI24 RGDS group, cells gathered in the defect and the tissue was repaired as 2, 4, 8 and 12 weeks after the transplantation. On the other hand, as shown in FIG. 7, no clear state of repair was observed in the control group as compared with the KI24RGDS group. This is clear also from the results of Ishida score shown in FIG. 8 and the R/D ratio shown in FIG. 9, and the Ishida score and R/D ratio were higher in the KI24RGDS group than in the control group at any stage of 2, 4, 8 and 12 weeks after transplantation, suggesting that tissue repair is progressing.

Next, the process of tissue regeneration by transplantation of the peptide hydrogel into the meniscus defect will be described with reference to FIGS. 10A to 10C. FIG. 10A is a HE-stained photograph of the meniscus tissue at 2 weeks after transplantation of the KI24RGDS group, and FIG. 10B is a safranin O-stained photograph. A model diagram for facilitating understanding is shown in FIG. 10C. As shown in FIGS. 10A to 10C, when the defect is filled with the peptide hydrogel, the peptide hydrogel serves as a scaffold, and cells migrate from the tissue adjacent to the defect along the periphery of the peptide hydrogel. It is considered that, as a result, cells that can differentiate into meniscus tissue gather in the defect and regenerate the tissue.

From the above results, the peptide hydrogel according to the present example has high mechanical strength, can be maintained in the defect when transplanted into the tissue defect of the joint tissue, and can exhibit an excellent effect of promoting tissue regeneration at the detect, thus, is extremely useful for regenerative therapy for joint diseases.

## Claims

1. A pharmaceutical composition for treating joint diseases comprising a peptide hydrogel constituted of a self-assembled peptide, wherein the peptide contains a β-hairpin structure constituted of β-sheet structures composed of alternating sequences of hydrophilic amino acids and hydrophobic amino acids, and a β-turn structure having biological activity.

2. The pharmaceutical composition according to claim 1, wherein the peptide hydrogel has a difference between the storage elastic modulus G' and the loss elastic modulus G" of 3900 Pa or more.

3. The pharmaceutical composition according to claim 1 or 2, wherein the peptide is composed of an amino acid sequence of SEQ ID NO: 1.

4. The pharmaceutical composition according to any one of claims 1 to 3, wherein the joint disease is meniscus injury.

5. A method for producing the pharmaceutical composition as described in any one of claims 1 to 4, which comprises
a step of preparing an aqueous solution containing said peptide, and
a step of adding phosphate buffered saline (PBS) to the aqueous solution to generate a peptide hydrogel.

6. The method according to claim 5, wherein the phosphate buffered saline is added to the aqueous solution so that the final concentration is 3 times the concentration (3 × PBS) or more.

7. The method according to claim 5 or 6, further comprising a step of adjusting the concentration of the phosphate buffered saline in order to adjust the optimum elastic modulus of the peptide hydrogel according to the site to which the pharmaceutical composition as described in any one of claims 1 to 4 is applied.


### [Sequence list]

SEQUENCE LISTING

<110>  EDUCATIONAL FOUNDATION OF OSAKA MEDICAL AND PHARMACEUTICAL UNIVERSITY,
       THE SCHOOL CORPORATION KANSAI UNIVERSITY

<120>  Pharmaceutical composition for articular disease and method for
manufacturing the same

<130>  CP02110

<160>  1

<170>  PatentIn version 3.5

<210>  1
<211>  24
<212>  PRT
<213>  Artificial Sequence

<220>
<223>  Peputide including strand and turn

<400>  1
Ile Lys Ile Lys Ile Lys Ile Lys Ile Lys Arg Gly Asp Ser Lys Ile
1               5                   10                  15

Lys Ile Lys Ile Lys Ile Lys Ile
            20